# EUROPEAN PATENT APPLICATION

(11) **EP 2 772 540 A1**
(43) Date of publication of application: **03.09.2014**
(21) Application number: 11874546.2
(22) Date of filing: 27.10.2011
(51) Int. Cl.: C12N 15/11, C12Q 1/68, A61K 31/7088, A61P 19/02

(54) **METHOD FOR DETECTING SPECIFIC SINGLE NUCLEOTIDE POLYMORPHISM RELATED TO ANKYLOSING SPONDYLITIS AND KIT THEREFOR**

(71) Applicant: Gu, Jieruo, Guangzhou, Guangdong 510630 (CN)
(72) Inventor: Gu, Jieruo, Guangzhou, Guangdong 510630 (CN)
(74) Representative: Decamps, Alain René François
(86) International application number: PCT/CN2011/081361
(87) International publication number: WO 2013/060005

(57) **Abstract**

The present invention provides a nucleotide fragment of pathogenic single nucleotide polymorphism associated with ankylosing spondylitis, identified following genome-wide correlation analysis of the ankylosing spondylitis patient and healthy control population in large sample by an intensive genome-wide SNP chip. The nucleotide fragment is located at the site of rs17095830, wherein for the ankylosing spondylitis patients, the base as G is significantly more frequent than that of the healthy control; and the base as A at the site is significantly less than the healthy control. The base at the site of rs17095830 as G may be effective to aid diagnosis of the AS disease. Provided herein are also a method and kit for detecting the corresponding pathogenic single nucleotide polymorphism associated with ankylosing spondylitis, which enables early aided specific diagnosis of ankylosing spondylitis and has broad application prospects.

SEQUENCE LISTING

<110> Gu, Jierou

<120> METHOD AND KIT FOR DETECTING SPECIFIC SINGLE NUCLEOTIDE POLYMORPHISM
ASSOCIATED WITH ANKYLOSING SPONDYLITIS

<140> CNWO201306005

<160> 3

<170> BiSSAP 1.2

<210> 1
<211> 30
<212> DNA
<213> Artificial Sequence

<220>
<221> source
<222> 1..30
<223> /mol_type="unassigned DNA"
      /note="amplification primer"
      /organism="Artificial Sequence"

<400> 1
acgttggatg cattcccatc taccagagtg                                        30


<210> 2
<211> 30
<212> DNA
<213> Artificial Sequence

<220>
<221> source
<222> 1..30
<223> /mol_type="unassigned DNA"
      /note="amplification primer (sense)"
      /organism="Artificial Sequence"

<400> 2
acgttggatg ggtttcagag cagtaaagtg                                        30


<210> 3
<211> 22
<212> DNA
<213> Artificial Sequence

<220>
<221> source
<222> 1..22
<223> /mol_type="unassigned DNA"
      /note="extension primer"
      /organism="Artificial Sequence"

<400> 3
ttccctacca gagtgtgtct ca                                                22

## Description

### BACKGROUND OF THE INVENTION

### Field of Invention

The present application relates to a method and kit for detecting a specific single nucleotide polymorphism (SNP) associated with ankylosing spondylitis, which is within the field of biomedical detection.

### Related Art

Ankylosing spondylitis (AS) is a subtype of spondyloarthropathies (SpA), with its main clinical manifestations being manifestations on medial axis such as inflammatory lumbago, morning stiffness and limited spinal activity, or/and with peripheric arthritis, inflammation of muscle tendon terminal and ligament appendifer, and spine stiffness or deformity occurring at the advanced stage of the disease. In prior studies, it was indicated that genetic factors played a greater role in pathogenesis of AS; in previous studies on twins, it was shown that the high and consistent morbidity among the monozygotic and dizygotic twins implicated 90% contribution of the genetic factors to pathogenesis of AS.

In the process of studies on AS pathogenic genes, HLA-B27 was first considered as an important gene associated with familial aggregation of AS inheritance. However, in terms of genic risks, it was suggested that HLA-B27 comprised only 20-30% of the total genetic risk, while overall MHC comprised 40-50% of the total genetic risk. MHC, also called a major histocompatibility complex, is a group of close linkage gene clusters encoding a major histocompatibility antigen, present in a certain chromosome of vertebrates and correlating with immune response, immunoregulation, graft rejection etc. It was indicated in studies that, for monozygotic twins, consistency of HLA-B27 had a probability of 63%, and for bizygotic twins, consistency of HLA-B27 had a probability of 23%. It also was indicated in studies that, the HLA-B27 positive, first-level relatives of AS patients had 6-16 times the probability of HLA-B27 positive individuals without family history, demonstrating that non-HLA-B27 familial factors also played an important role in development of diseases.

In recent years, the genome-wide association studies have been increasingly and widely applied to seek the susceptible genes, providing a creative ideal for studying the mechanisms of generation and development of ankylosing spondylitis, and with full scanning and analyses on genome-wide SNP chips, the SNP susceptible to and associated with ankylosing spondylitis was sought, probing into the pathogenic genes of or genes susceptible to ankylosing spondylitis.

The single nucleotide polymorphism (SNP) refers to the DNA sequence polymorphism resulted from a variation of single nucleotide in genome level, which is one of the most common genetic variations in human and comprises more than 90% of all known polymorphisms. SNP is widely present in human genome, with one SNP per 500 - 1000 base pairs on average and its total number estimated to be up to 3 million or even more. The polymorphism reflected by SNP only confines itself to a single base variation resulted from transition or transversion of single base or insertion or deletion of base. Since in genomic DNA, a variation might occur to any base, SNP might be present in the gene sequence or non-coding sequence outside of the gene. In general, although due to falling within the exon, the coding SNP (cSNP) is in small numbers and is 5 times lower than the variation of sequences around it, it is of far reaching importance, and attracts more attention on the cSNP studies. In terms of effects on the biological genetic traits, cSNP can be classified into 2 types: a synonymous cSNP, i.e. the SNP by which alteration of the coding sequence has not effects on the amino acid sequence of protein translated by it, and the mutated bases or codons are the same in meaning as the un-mutated bases or codons; a non-synonymous cSNP, which means that alteration of the base sequence allows the protein sequence translated from it as a template to alter, having an effect on the function of the protein and directly leading to changes of biological traits, with the non-synonymous cSNP comprising almost half of the cSNP. In addition, the SNP may be used as the genetic marker aiding in seeking and identifying the susceptible gene.

In 2007, with full scanning on do-it-yourself intensive SNP chip and verification for AS patients and healthy volunteers as control population in large sample, Brown et. al. pointed out that it was possible for both IL23R and ARTS1 genes to be the new genes susceptible to AS; subsequently, ARTS1 gene was verified in the research agencies of Korea and China, finding that there was a link between ARTS1 and AS; moreover, IL-23R was verified in the research agencies of China, finding that there was no link between IL-23R and AS, suggesting that there might be differences among various populations. Furthermore, although in the studies of Brown above in 2007, the intensive SNP chip was employed, full scanning of all of the genome was not carried out on the intensive SNP chip customized on positions of interest only, leading to skip on the regions uncovered by the chip or insufficient to be intensively and fully scanned by the SNP marker. Upon that, in 2010, full scanning on the Illumina HumHap 370 chip and verification were carried out by Brown et. al. on, as the European ethnic progeny, the Australian, Britain and North American AS patients and healthy volunteers as control population, finding that besides that it was possible for both ARTS1 and IL-23R genes to be those susceptible to AS in the previous studies, there was also a possible relationship between ANTXR and AS, and it suggested that there were SNP associated with AS in 2p15 and 21q22.

With rapid development of the chip technology, the Illumina HumHap 370 chip was followed by Illumina Human 1M-duo, Illumina Human610-Quad and Illumina OmniExpress chips of new generation, providing more intensive SNP markers and extensive coverage area. In view of above-mentioned background of study, the association analysis, i.e. genome-wide association study (GWAS), with full scanning on the Illumina Human 1M-duo, Illumina Human610-Quad and Illumina OmniExpress chips for Chinese Han nationality AS patients and healthy volunteers as control population, is invented to probe if the genes susceptible to and associated with AS would be found.

Since ankylosing spondylitis is the more common disease, with long course of disease and tendency to disability, earlier diagnosis and treatment should be striven for, especially for 16-25-year old young people. In order to promote earlier diagnosis of ankylosing spondylitis, there is an urgent need in the art to find out the SNP closely associated with ankylosing spondylitis, and provide the method and kit specific for detecting ankylosing spondylitis.

To date, for diagnosis of ankylosing spondylitis, there was no report about the specific indicators of laboratory diagnosis and corresponding methods as well as kits for specific diagnosis of the disease, except for using the clinical symptoms and imaging data to aid judgment.

### SUMMARY OF THE INVENTION

The present application provides an enhanced specific detection method and a kit therefor, against disadvantage as current absence of the specific detection method and kit therefor in early diagnosis of AS.

In the insight and widespread studies, by the genome-wide correlation analysis of ankylosing spondylitis patients and healthy control population of large sample with the intensive genome-wide SNP chips, it was first found and confirmed that SNP rs17095830 is significantly correlated with ankylosing spondylitis, wherein the SNP base is of risk type for G and is of protection type for A, *P* = 1.63x10⁻⁸ (this finding has been adopted in a journal of international authority, Nature Genetic and will be published soon after), therefore SNP rs17095830 may be used as the specific SNP for detecting ankylosing spondylitis, and becomes the important indicator of early aided diagnosis of ankylosing spondylitis, and based on this, the present invention is achieved.

It is one of the objects for the present application to provide a nucleotide fragment of pathogenic single nucleotide polymorphism (SNP) of ankylosing spondylitis, located at the site of SNP rs17095830; for AS patients, the base as G (guanine nucleotide) is significantly more frequent than that of the health controls, in contrast the base as A (adenosine nucleotide) is significantly less frequent than that of the healthy controls, i.e. the base at rs17095830 as G may be effective to aid diagnosis of the AS disease.

It is one of the objects for the present application to provide a method for early aided diagnosis of ankylosing spondylitis by obtaining the nucleotide sequence from the sample to be tested, detecting if the base at the site of rs17095830 is G and judging the probability of suffering ankylosing spondylitis to significantly increase if the base at the site is G.

Further, the sample includes blood, body fluid or tissue.

Further, the method includes the following steps:
1. Extracting DNA from the sample to be tested;
2. With the DNA as a template, designing a PCR primer for coding regions adjacent to the rs17095830 base, for PCR reaction, to give a PCR reaction product;
3. Sequencing for the bases of the PCR reaction product; and
4. Comparing the resulted base sequence with a normal gene sequence, to identify if the base at the site of rs17095830 is G.

Further, in the method above, obtaining the nucleotide sequence from the sample to be tested also may include fluorescent quantitative PCR, denaturing high performance liquid chromatography, restrictive fragment length polymorphism, or ligase detection and sequencing.

It is one of the objects for the present invention to provide a method for detecting the specific single nucleotide polymorphism above of ankylosing spondylitis, including the following steps:
1. Primer designing:
   Genotyping Tools and MassARRAY Assay Design software from Sequenom Corp. can be used to design a primer for PCR amplification and a primer for single base extension at the site of rs17095830;
2. Extracting DNA from tissue, cell and blood samples:
   The TIANamp Blood DNA kit for extracting blood genomic DNA or NucleoSpin Tissue (MN) can be used to extract DNA from tissue, cell or blood samples, and perform quantitative measurement with a spectrophotometer and running on agarose gel for electrophoresis, and the qualified DNA would be adjusted to the concentration of 50 ηg/L and stored at -20°C until use.
3. PCR amplification:
   The multiplex PCR amplification can be used, with the total volume of 5 µl per reaction comprising 10 ηg template DNA, 0.5 U Hotstar Taq, 0.5 pmol/strand amplification primer and 0.1 µl 25 mM dNTP, at the reaction conditions of 94°C for 4 minutes; 94°C for 20 seconds, 56°C for 30 seconds, 72°C for 1 minute, in total of 45 cycles; 72°C for 3 minutes; and 4°C ∞;
4. Purification of PCR product:
   The PCR reaction product is treated with 0.5 U shrimp alkaline phosphatase (SAP) for removal of free dNTP from the system, with a reaction system of 7 µl comprising 5 µl PCR product and 2 µl SAP mixture (0.5 U SAP, 0.17 µl buffer) and the reaction procedure of 37°C for 20 minutes, 85°C for 5 minutes and 40°C ∞;
5. Single base extension:
   The reaction system with total volume of 9 µl comprises 7 µl PCR product from SAP treatment, 0.804 µl primer hybrid for each extension reaction, 0.041 µl iPLEX enzyme and 0.2 µl extension hybrid, and the reaction procedure comprises 94°C for 30 seconds; 94°C for 5 seconds; 52°C for 5 seconds, 80°C for 5 seconds in 5 cycles; back to 94°C for 5 seconds in total of 40 cycles; 72°C for 3 minutes and 4°C ∞;
6. Purification on resin:
   Each of extension reaction products can be purified on 6 mg Clean Resin;
7. Spotting on chip and mass spectrography:
   The purified product to be tested is transferred to 384 well SpectroCHIP (Sequenom) chip with the matrix-assisted laser desorption/ionization-time of flight (MALDI-TOF) mass spectrography, and the tested results are classified and output with TYPER 4.0 software (sequenom); and
8. The single base detected at the site of rs17095830 is analyzed, and if the base is guanine nucleotide G, it is indicated that the probability of suffering ankylosing spondylitis is significantly increased.

It is one of the objects for the present application to provide the kit for detecting the specific single nucleotide polymorphism of ankylosing spondylitis, comprising: the PCR amplification primer designed for the regions adjacent to the rs17095830 base, the extension primer, the DNA-polymerase, the de-ionized water, dNTP, MgCl2, the PCR buffer, SAP and Clean Resin.

It is one of the objects for the present application to provide an application of the specific single nucleotide polymorphism of ankylosing spondylitis in the diagnosis and treatment of the associated susceptible or pathogenic genes, i.e. an application of the base at the site of rs17095830 being of risk type for G and of protection type for A in the treatment of ankylosing spondylitis.

Further, for the susceptible or pathogenic gene associated with the specific single nucleotide polymorphism of ankylosing spondylitis, the base at the site of rs17095830 is guanine nucleotide G.

The application includes an application of the susceptible or pathogenic genes associated with the specific single nucleotide polymorphism (SNP) of ankylosing spondylitis in the preparation of medicament for treatment of ankylosing spondylitis.

In particular, the application includes preparation of therapeutic medicament by intervening the functions of the susceptible or pathogenic genes at rs17095830 associated with ankylosing spondylitis, including translation, transcription and protein synthesis processes, for improvement of bone metabolic disorder and immune inflammatory response in the body of the patients, for the purpose of therapy.

### DETAILED DESCRIPTION OF THE INVENTION

In the following, the method for detecting susceptibility to ankylosing spondylitis and kit therefor described in the present application will be exemplified, in conjunction with the specific embodiments, for the purpose of a better understanding of the description of the present application, rather than limitation thereto. As a matter of fact, it is intended that any of modifications of the process steps, reagents and reaction conditions, and the kit by the same or similar theory for the purpose of essentially same effects, will fall into the scope of the present application.

### Embodiment 1

### Study 1 on correlation of SNP rs17095830 with occurrence of ankylosing spondylitis

### 1.1 Subject of study

1837 sporadic AS patients were available for the study, mainly comprising those who had primarily visited the Subsidiary Third Hospital, Zhongshan University, as outpatients or inpatients, in 2005-2009, diagnosed to meet the revised New York standard 1984 by specialists skilled in the rheumatology according to clinical manifestation and imaging results. After being told about the purpose of the study, each of AS patients agreed for blood sampling for the study and signed the Informed Consent Form.

4231 healthy volunteers as control population primarily included those in Guangdong and Anhwei, and Chinese in Singapore, without AS symptoms or medical history, who also agreed for blood sampling for the study after being told about that and signed the Informed Consent Form.

At the time of case recruitment, the cases and healthy volunteers as control population were recorded for background, including: sex, age and native place. 4 ml whole blood was sampled into an EDTA tube and brought back to the laboratory for extracting DNA. In addition to blood sampling, each of AS patients and healthy volunteers as control population was inquired in detail and recorded, especially for background (including: sex, age, onset age, course of disease) and clinic phenotypes (including: sausage fingers, hip joint involvement, peripheric arthritis and inflammatory lumbago) of AS patients. The healthy volunteers as control population were inquired in detail for past medical history, including respiratory system, cardiovascular system, endocrine system, blood system, digestive system, skeletal muscle system, nervous system etc.; in addition to highlighting the rheumatism and immune medical history, whether there was the past medical history of lumbago etc. was inquired.

### 1.2 Laboratory procedures and results

### 1.2.1 Extraction of DNA

In the study, the TIANamp Blood DNA kit for extracting blood genomic DNA was used to extract DNA from the samples of the subject peripheric blood, which comprised: a cytolysis solution, a buffer GS, a buffer GB, a buffer GD, a wash solution PW, an elution buffer TB, a protease K, an adsorbent column CB3, a collection tube (2 ml) and 1.5 mL sterile collection tube.

### 1.2.2. The intensive genome-wide SNP chip used in the study

The Illumina HuamHap 610-Quad SNP, Illumina Human 1M-duo and Illumina OmniExpress chips were used in the study for full scanning of the genome-wide SNP of samples, which were introduced briefly as follows;

### 1. Illumina HuamHap 610-Quad SNP chip

For this chip, 4 samples are detected in parallel on one chip, significantly increasing output information of samples and reducing errors in laboratory operation. In the chip, the contents of HumanHap550 chip are broadly adopted, and there are the additionally increased 100,000 genetic markers. The genome-wide information embodied by the Human 610-quad is authoritative for the known and recently reported CNV region. The SNPs of Illumina HuamHap 610-Quad SNP chip are uniformly distributed at the density of about 5 kb on the genome. For the CNV regions (the fragment replication region and the genomic regions without SNP) of high polymorphism in the genome, some specific targets are designed for the Illumina HuamHap 610-Quad SNP chip in the study.

### Illumina Human 1M-duo chip.

The chip comprises information about more than one million probes. The chip comprises the probes designed for the gene SNPs, label SNPs, Copy Number Variation (CNV), and other valuable genomic regions, in addition to some new sites, such as the SNP sites associated with diseases and the SNP sites of high density which allow some genomic coding regions to be selected.

### 2. Illumina OmniExpress chip

The OmniExpress chip provides high throughput of sample and full genomic contents and holds 12 samples, with over 700 thousand variation copies per sample in the chip and total of more than 8 million data points in a single chip. The marker on the OmniExpress is a portion of contents of the Illumina HumanOmni1-Quad, which is a preferred combination of SNP labels selected from all of three stages in the International HapMap Program, and has the top data quality and optimal contents, including full support to application of copy number variation (CNV).

### 1.2.3 Study steps

The genome-wide SNP typing was carried out by the three chips above for all of the sporadic AS cases and healthy volunteers as control. Subsequently, the correlation analysis was carried out, wherein the data was normalized for sex, age, and analytic results of main components respectively, and the SNPs having statistic significance, i.e. SNPs associated likely with disease were sought,

### 1.2.3.1 Sample preparation and genotyping

After extraction of DNA from the whole blood samples, all of the DNAs were normalized to the concentrated solution of 50 ng/ml. For typing by the high density SNP chips, genotyping of the samples was carried out by the chips with about 200 ng DNA according to the operational procedure recommended by Illumina, and data reading and genotype switchover were carried out with the Illumina Beadstudio. In the resulted data, the three chips shared 272386 SNPs, and moreover, by statistic analysis, it was predicted that 1083964 SNPs were available for the analysis.

### 1.2.3.2 Quality control (QC) for data analysis

In the study, the quality control and monitoring, including call rate <95%, minor allele frequency (MAF) <3%, and deviation from Hardy-Weinberg Equilibrium (HEW, P<10⁻⁶), were carried out on the AS cases and healthy volunteers as control population respectively, to remove the SNP with wrong typing as best as one can; after obtaining the genotype data of samples from full scanning, the heterozygosity was analyzed and calculated, and the data samples in the range of mean heterozygosity - 3 x SD to mean heterozygosity + 3 x SD were available for statistic analysis and the data samples out of this range were removed and excluded from further statistic analysis. Subsequently, these data were tested for genetic correlation by the PLINK software and the method based on identical by state (IBS), respectively, and for those repeats based on genetic analysis or a pair of samples having genetic relationship, one in which it has a lower call rate therein was removed. The population stratification in samples was tested by the principle component analysis (PCA) method and population data in HapMap database z, and the obvious outliers were removed. Finally, the intersection was obtained from these independent data.

### 1.2.3.3 Statistic analysis

For analysis of the data from full scanning, in view of population outlying and stratification to a certain extent, before such analysis, the EIGENSTRAT software was first utilized to analyze all the samples, i.e. primary component analysis (PCA) of the samples, and provide 3 primary components and analyze whether cluster of the samples was present among these 3 components, to identify whether population discrete degree of these samples was required to use as the parameter to be normalized. Next, the Quantile-Quantile plot was drawn on a basis of the data of these samples, wherein before plotting, it was required to normalize all the factors required for normalization, including sex, age (if there was statistic difference between the sporadic case and healthy volunteers as control population), and λ_{GC} value obtained as a result of presence of cluster of primary components after PCA analysis above. For analysis of the resulted data, the chi-square testing was used by the software to test allele frequency of each of the sporadic cases and gene frequency of the healthy volunteers as control population, to give the chi-square value A, and λ_{GC} = Amean/0.456, wherein the closer λ_{GC} is to 1, the higher for availability of the samples; in the case of deviation, it was suggested that there might be both stratification for the samples, i.e. discrete obviousness for population, and the SNPs associated with the disease risk.

After the analysis above, if it was suggested for the results from the PCA analysis that there was stratification among the primary components, i.e. difference between discrete degrees of populations, the discrete degrees of difference were normalized as covariant. In addition, the logistic regression analysis was carried out by the PLINK software with age and sex (if there was difference between the sporadic case and healthy volunteers as control population) as covariant, to seek the SNP sites associated with the disease. The Haploview (v4.1) software was used to display the analytic results, i.e. SNP chromosome sites and the Manhattan plot represented by its -log10P.

### 1.2.3.4 SNP prediction and linkage disequilibrium pattern analysis

Prediction of SNPs in the three chips for analysis was achieved by the IMPUTE2 software (version number: 2.1.0), to maximize the coverage of the chip for chromosome.

### 1.2.4 Results

There was a significant difference between the 1837 AS cases and 4231 healthy volunteers as control (P value = 1.91x10⁻⁵), i.e. significant correlation with ankylosing spondylitis, and the SNP base was of risk type for G and of protection type for A.

From the results above, it is indicated that in SNP rs17095830 at the site of 44061175 in chromosome 12 of 36.3Genome Buildde Human Genome Map, base A→G alteration allows susceptibility to AS to enhance, and the allelotype G is significantly correlated with development of AS.

### Embodiment 2

### Study 2 on correlation of SNP rs17095830 with occurrence of ankylosing spondylitis

### 2.1 Subject of study

2205 sporadic AS patients were available for the study, mainly consisting of those who had visited the Subsidiary Third Hospital, Zhongshan University, as outpatients or inpatients, in 2005-2009, and the samples from other hospitals, diagnosed to meet the revised New York standard 1984 by specialists skilled in the rheumatology according to clinical manifestation and imaging results. After being told about the purpose of the study, each of AS patients agreed for blood sampling for the study and signed the Informed Consent Form.

3702 healthy volunteers as control population primarily included those recruited by the Subsidiary Third Hospital, Zhongshan University, and Tumor Prophylaxis and Treatment Center, Zhongshan University, without AS symptoms or medical history, who also agreed for blood sampling for the study after being told about that and signed the Informed Consent Form.

### 2.2 Laboratory procedures and results

### 2.2.1 Extraction of DNA

In the study, the TIANamp Blood DNA kit for extracting blood genomic DNA was used to extract DNA from the samples of the subject peripheric blood, which comprised: the cytolysis solution, the buffer GS, the buffer GB, the buffer GD, the wash solution PW, the elution buffer TB, the protease K, the adsorbent column CB3, the collection tube (2 ml) and 1.5 mL sterile collection tube.

### 2.2.2 Study steps

### 2.2.2.1 Primer designing

The primers, including the primer for amplification and the primer for extension, were designed by iPLEX® Gold snp genotyping in Massarray® DNA mass spectroscopy array gene analytical system, Sequenom Corp. The information about the primers for SNP rs17095830 were as follows:

To distinguish between the primer for amplification and the primer for extension, and enhance PCR efficiency and mass spectroscopy, a marker having ten bases was added to the primer for amplification at 5' end, i.e. 5'-ACGTTGGATG-3'. Amplification, extension and analysis were carried out under the following laboratory conditions.

| SNP_ID | Antisense strand of the primer for amplification (SEQ ID NO. 1) | Sense strand of the primer for amplification (SEQ ID NO. 2) | Size of fragment to be amplified | Lysis temperature | The primer for extension (SEQ ID NO. 3) |
|---|---|---|---|---|---|
| rs17095830 | ACGTTGGATG CATTCCCATC TACCAGAGTG | ACGTTGGATG GGTTTCAGAG CAGTAAAGTG | 98 | 50 | TTCCCTACCAG AGTGTGTCTCA |

| | | | | | |
|---|---|---|---|---|---|
| 2.2.2.2 Genomic DNA amplification (polymerase chain reaction PCR): | | | | | |

2.2.2.2.1 The reaction system as follows:

| Reagent | Concentration (in 5 µl) | Volume (µl per well) | Volume (µl, 384 well) |
|---|---|---|---|
| De-ionized water | NA | 1.9 | 875.5 |
| 10x PCR Buffer with 20 mM MgCl₂ | 1x (2 mM MgCl₂) | 0.5 | 230.4 |
| MgCl₂ (25 mM) | 2 mM | 0.4 | 184.3 |
| dNTP mix (25 mM each) | 500 µM | 0.1 | 46.1 |
| Primer (500 nM each) | 100 nM | 1.0 | 460.8 |
| PCR enzyme (5 U/µL) | 0.5 U/rxn | 0.1 | 46.1 |
| Total volume | | 4.0 | 1843.2 |

### 2.2.2.2.2 PCR steps:

To the 384 well plate, 1 µl DNA and then 4 µl reaction system above were added per well, centrifuged at 1000 rpm for 1 minute, mixed homogeneously, and the amplification procedure was carried out as follows: pre-denaturing at 94°C for 4 minutes, 94°C for 20 seconds, 56°C for 30 seconds, 72°C for 1 minute in total of 45 cycles, 72°C for 3 minutes, and cooling at 4°C.

### 2.2.2.3 Mass spectrography:

The matrix-assisted laser desorption/ionization-time of flight mass spectrography (MALDI-TOF) is characterized in that initially the target sequence is amplified by PCR, and then the primer for extension specific to the snp sequence is added for extension of 1 base at the SNP site. The prepared sample analyte is co-crystallized with the chip matrix, and the crystal is placed into a vacuum tube of the mass spectrometer, followed by strong laser excitation in transient nanoseconds (10⁻⁹ s). The matrix molecules absorb energy under radiation, leading to energy storage and quick generation of heat, allowing the matrix crystal to sublimate and the nucleic acid molecules to be desorbed and shift to the meta-stable ions which are mostly the mono-charge ions. These mono-charge ions are imparted with same kinetic energy in the accelerating electric field, which further are separated in a non-electric field drift region according to its mass-to-charge ratio and fly in the vacuum tubule to the detector. The ions generated by MALDI are detected by a Time-of-Flight (TOF) detector, and the smaller the mass of ion is, the faster the arrival is. The genotype of SNP in the PCR reaction products above is detected immediately and conveniently by this method.

### 2.2.2.4 Statistic analysis

The quality control and monitoring were carried out on the AS cases and healthy volunteers as control population, wherein the samples, including call rate < 95%, minor allele frequency < 3% and deviation from Hardy-Weinberg Equilibrium (P < 10⁻⁶), were excluded for further analysis. By quality control, 2,100 patients and 3,496 healthy controls went through for further statistic analysis. The logistic regression analysis was carried out on the tested samples and all the samples by the PLINK software and R method, to seek the SNP sites associated with disease. The linkage map in the region where the SNP sites were located was plotted by the Haploview (v4.1) software.

### 2.2.3 Results

There was a significant difference between the 2205 AS cases and 3702 healthy volunteers as control (P value = 1.27×10⁻⁴), i.e. significant correlation with ankylosing spondylitis, and the SNP base was of risk type for G and of protection type for A.

From the results above, it is indicated that in SNP rs17095830 at the site of 44061175 in chromosome 12 of 36.3Genome Buildde Human Genome Map, base A→G alteration allows susceptibility to AS to enhance, and the allelotype G is significantly correlated with development of AS.

### Embodiment 3

### 3.1.1 Subject of study

The study was directed to those AS and healthy control populations in Embodiment 1 and Embodiment 2, diagnosed to meet the revised New York standard 1984 by specialists skilled in the rheumatology according to clinical manifestation and imaging results. After being told about the purpose of the study, each of AS patients agreed for blood sampling for the study and signed the Informed Consent Form. Finally, a total of 3937 AS patients and 7727 healthy volunteers as control were included in the study.

### 3.2.2 Extraction of DNA

For the samples to be detected, the TIANamp Blood DNA kit for extracting blood genomic DNA was used to extract DNA from the samples of the subject peripheric blood, which comprised: the cytolysis solution, the buffer GS, the buffer GB, the buffer GD, the wash solution PW, the elution buffer TB, the protease K, the adsorbent column CB3, the collection tube (2 ml) and 1.5 mL sterile collection tube.

### 3.2.3 Laboratory steps

The data about SNP rs17095830, under data quality control, from all of the AS and healthy controls in Embodiment 1 were extracted, while the data about SNP rs17095830, under data quality control, from all of the AS and healthy controls in Embodiment 2 were extracted; both were processed and put back into the same database for analysis. For statistic analysis, the logistic regression analysis was carried out by the PLINK software on the data of SNP rs17095830 of all the samples, with such disturbing factors as age, sex and the like being normalized, thus determining if there was a correlation between the SNP rs17095830 data and disease.

### 3.3 Laboratory results:

It was indicated from the results that there was a significant difference in SNP rs17095830 between the AS patients and healthy volunteers as control, P value = 1.63x10⁻⁸, i.e. the base at the site of SNP rs17095830 was significantly correlated with ankylosing spondylitis, wherein the SNP base is of risk type for G and of protection type for A. The results were consistent with both studies above.

### Embodiment 4

### 4.1.1 Subject of study

All of patients and relatives were those intended for detecting if they carried the genes susceptible to ankylosing spondylitis.

### 4.2.2 Extraction of DNA

For the samples to be detected, the TIANamp Blood DNA kit for extracting blood genomic DNA was used to extract DNA from the samples of the subject peripheric blood, which comprised: the cytolysis solution, the buffer GS, the buffer GB, the buffer GD, the wash solution PW, the elution buffer TB, the protease K, the adsorbent column CB3, the collection tube (2 ml) and 1.5 mL sterile collection tube.

### 4.2.3 Laboratory steps

### 4.2.3.1 PCR steps:

To the plate to be tested, 1 µl DNA and then 4 µl reaction system above were added per well, centrifuged at 1000 rpm for 1 minute, mixed homogeneously, and the amplification procedure was carried out as follows: pre-denaturing at 94°C for 4 minutes, 94°C for 20 seconds, 56°C for 30 seconds, 72°C for 1 minute in total of 45 cycles, 72°C for 3 minutes, and cooling at 4°C.

### 4.2.3.2 Mass spectrography:

After purification on resin, the prepared sample analyte was co-crystallized with the chip matrix, and the crystalline was placed into the vacuum tube of mass spectrometer followed by strong laser excitation with transient nanoseconds (10⁻⁹ s), thus immediately and conveniently detecting the genotype of SNPs in the PCR reaction product above.

### 4.3 Laboratory results:

### It is of risk type when the detected result is G and is of protection type when the detected result is A.

The kit comprises: the primer for PCR amplification for the region adjacent to the rs17095830 base, the primer for extension, the DNA-polymerase, de-ionized water, dNTP, MgCl₂, PCR buffer, SAP and clean resin, wherein the primer for amplification and the primer for extension are shown in the table below:

| SNP_ID | Antisense strand of the primer for amplification (SEQ ID NO. 1) | Sense strand of the primer for amplification (SEQ ID NO. 2) | Size of fragment to be amplified | Lysis temperature | The primer for extension (SEQ ID NO. 3) |
|---|---|---|---|---|---|
| rs17095830 | ACGTTGGATG CATTCCCATC TACCAGAGTG | ACGTTGGATG GGTTTCAGAG CAGTAAAGTG | 98 | 50 | TTCCCTACCAG AGTGTGTCTCA |

The detecting method comprises the steps of:
1. Primer designing:
   Genotyping Tools and MassARRAY Assay Design software from Sequenom Corp. are used to design the primer for PCR amplification and the primer for single base extension at the SNP site to be detected;
2. Extracting DNA from tissue, cell and blood samples:
   The TIANamp Blood DNA kit for extracting blood genomic DNA or NucleoSpin Tissue (MN) is used to extract DNA from tissue, cell or blood samples, and perform quantitative measurement with the spectrophotometer and running on agarose gel for electrophoresis, and the qualified DNA would be adjusted to the concentration of 50 ηg/L and stored at -20°C until use.
3. PCR amplification:
   The multiplex PCR amplification is used, with the total volume of 5 µl per reaction comprising 10 ηg template DNA, 0.5 U Hotstar Taq, 0.5 pmol/strand primer for amplification and 0.1 µl 25 mM dNTP, at the reaction conditions of 94°C for 4 minutes; 94°C for 20 seconds, 56°C for 30 seconds, 72°C for 1 minute, in total of 45 cycles; 72°C for 3 minutes; and 4°C ∞;
4. Purification of PCR product:
   The PCR reaction product is treated with 0.5 U shrimp alkaline phosphatase (SAP) for removal of free dNTP from the system, with the reaction system of 7 µl comprising 5 µl PCR product and 2 µl SAP mixture (0.5 U SAP, 0.17 µl buffer) and the reaction procedure of 37°C for 20 minutes, 85°C for 5 minutes and 40°C ∞;
5. Single base extension:
   The reaction system with total volume of 9 µl comprises 7 µl PCR product from SAP treatment, 0.804 µl primer hybrid for each extension reaction, 0.041 µl iPLEX enzyme and 0.2 µl extension hybrid, and the reaction procedure comprises 94°C for 30 seconds; 94°C for 5 seconds; 52°C for 5 seconds, 80°C for 5 seconds in 5 cycles; back to 94°C for 5 seconds in total of 40 cycles; 72°C for 3 minutes and 4°C ∞;
6. Purification on resin:
   Each of extension reaction products is purified on 6 mg Clean Resin;
7. Spotting on chip and mass spectrography:
   The purified product to be tested is transferred to the 384 well SpectroCHIP (Sequenom) chip with matrix-assisted laser desorption/ionization-time of flight (MALDI-TOF) mass spectrography, and the tested results are classified and output with TYPER 4.0 software (sequenom); and
8. The single base detected at the site of rs17095830 is analyzed, and if the base is guanine nucleotide G, it is indicated that the probability of suffering ankylosing spondylitis is significantly increased.

## Claims

1. A nucleotide fragment of pathogenic single nucleotide polymorphism associated with ankylosing spondylitis, wherein the nucleotide fragment is located at the site of rs17095830, and for a ankylosing spondylitis patient, the base as G is significantly more frequent than that of healthy control; in contrast, the base as A at the site is significantly less frequent than that of healthy control.

2. A method for early aided diagnosis of ankylosing spondylitis, wherein the method is carried out by obtaining a nucleotide sequence from a sample to be tested, detecting if a base at the site of rs17095830 is G and judging a probability of suffering ankylosing spondylitis to increase if the base at the site is G.

3. The method according to claim 2, wherein the sample comprises blood, body fluid or tissue.

4. The method according to claim 2, wherein the method comprises the following steps:
1) extracting DNA from the sample to be tested;
2) with the DNA as a template, designing a PCR primer for coding regions adjacent to the rs17095830 base, for PCR reaction, to give a PCR reaction product;
3) sequencing for the bases of the PCR reaction product; and
4) comparing the resulted base sequence with a normal gene sequence, to identify if the base at the site of rs17095830 is G.

5. The method according to claim 2, wherein the method for obtaining the nucleotide sequence from the sample to be tested comprises the fluorescent quantitative PCR, the denaturing high performance liquid chromatography, the restrictive fragment length polymorphism, the ligase detection or the DNA fragment sequencing.

6. A method for detecting a specific single nucleotide polymorphism of ankylosing spondylitis, wherein the method comprises the following steps:
1) Primer designing:
Genotyping Tools and MassARRAY Assay Design software from Sequenom Corp. are used to design a primer for PCR amplification and a primer for single base extension at the site of rs17095830;
2) Extracting DNA from tissue, cell and blood samples:
The TIANamp Blood DNA kit for extracting blood genomic DNA or NucleoSpin Tissue (MN) is used to extract DNA from tissue, cell or blood samples, and perform quantitative measurement with a spectrophotometer and running on agarose gel for electrophoresis, and the qualified DNA would be adjusted to the concentration of 50 ηg/L and stored at -20°C until use;
3) PCR amplification:
The multiplex PCR amplification is used, with the total volume of 5 µl per reaction comprising 10 ηg template DNA, 0.5 U Hotstar Taq, 0.5 pmol/strand primer for amplification and 0.1 µl 25 mM dNTP, at the reaction conditions of 94°C for 4 minutes; 94°C for 20 seconds, 56°C for 30 seconds, 72°C for 1 minute, in total of 45 cycles; 72°C for 3 minutes; and 4°C ∞;
4) Purification of PCR product:
The PCR reaction product is treated with 0.5 U shrimp alkaline phosphatase (SAP) for removal of free dNTP from the system, with the reaction system of 7 µl comprising 5 µl PCR product and 2 µl SAP mixture (0.5 U SAP, 0.17 µl buffer) and the reaction procedure of 37°C for 20 minutes, 85°C for 5 minutes and 40°C ∞;
5) Single base extension:
The reaction system with total volume of 9 µl comprises 7 µl PCR product from SAP treatment, 0.804 µl primer hybrid for each extension reaction, 0.041 µl iPLEX enzyme and 0.2 µl extension hybrid, and the reaction procedure comprises 94°C for 30 seconds; 94°C for 5 seconds; 52°C for 5 seconds, 80°C for 5 seconds in 5 cycles; back to 94°C for 5 seconds in total of 40 cycles; 72°C for 3 minutes and 4°C ∞;
6) Purification on resin:
Each of extension reaction products is purified on 6 mg Clean Resin;
7) Spotting on chip and mass spectrography:
The purified product to be tested is transferred to the 384 well SpectroCHIP (Sequenom) chip with matrix-assisted laser desorption/ionization-time of flight (MALDI-TOF) mass spectrography, and the tested results are classified and output with TYPER 4.0 software (sequenom); and
8) the single base detected at the site of rs17095830 is analyzed and if the base is guanine nucleotide G, it is indicated that the probability of suffering ankylosing spondylitis is significantly increased.

7. A kit for detecting a specific single nucleotide polymorphism of ankylosing spondylitis, wherein the kit comprises: a primer for PCR amplification designed for the regions adjacent to the rs17095830 base, a primer for extension, a DNA-polymerase, de-ionized water, dNTP, MgCl₂, a PCR buffer, SAP and clean resin.

8. An application of the pathogenic single nucleotide polymorphism associated with ankylosing spondylitis described in claim 1 in the treatment of ankylosing spondylitis.

9. The application according to claim 7, wherein the application comprises an application of the susceptible or pathogenic genes associated with the specific single nucleotide polymorphism of ankylosing spondylitis in the preparation of medicament for treatment of ankylosing spondylitis.

10. The application according to claim 8, wherein for the susceptible or pathogenic genes associated with the specific single nucleotide polymorphism of ankylosing spondylitis, the base at the site of rs17095830 is guanine nucleotide.

11. The application according to claim 7, wherein the application comprises preparation of therapeutic medicament by intervening the functions of the susceptible or pathogenic genes at rs17095830 associated with ankylosing spondylitis, comprising translation, transcription and protein synthesis processes, for improvement of bone metabolic disorder and immune inflammatory response in the body of the patients, for the purpose of therapy.
